# EUROPÄISCHE PATENTANMELDUNG

(11) **EP 3 205 363 A1**
(43) Veröffentlichungstag der Anmeldung: **16.08.2017**
(21) Anmeldenummer: 17154502.3
(22) Anmeldetag: 03.02.2017
(51) Int. Cl.: A61M 5/24, A61M 5/315

(54) **VORRICHTUNG ZUR BESTIMMUNG DES FÜLLSTANDES IN EINER KARPULE**

(30) Priorität: 10.02.2016 AT 500862016
(71) Anmelder: AIT Austrian Institute of Technology GmbH, 1220 Wien (AT)
(72) Erfinder: Meindl, Michael, 2632 Wimpassing (AT); Lurf, Robert, 2640 Gloggnitz (AT); Beisteiner, Martin, 2853 Bad Schönau (AT)
(74) Vertreter: Wildhack & Jellinek

(57) **Zusammenfassung**

Die Erfindung betrifft eine Vorrichtung zur Bestimmung des Füllstandes in einer Karpule (2), wobei der Füllstand mittels zumindest einer auf zumindest einer Folie (3) angebrachten Elektrode (4) messbar ist, umfassend einen Karpulenhalter (10), der eine Ausnehmung (11) aufweist, in die die Karpule (2) eingebracht ist, wobei die Folie (3) im Zwischenbereich zwischen dem Karpulenhalter (10) und der Karpule (2) oder im Werkstoff des Karpulenhalters (10) angeordnet ist.

Erfindungsgemäß ist vorgesehen, dass der Karpulenhalter (10) im Bereich zumindest einer Elektrode (4) einen, insbesondere vorgespannten, flexiblen Bereich (12) und/oder ein flexibles Element (18) aufweist, wobei der flexible Bereich (12) oder das flexible Element (18) die Folie (3), zumindest im Bereich der Elektrode (4), in Richtung der Karpule (2) andrückt.

## Beschreibung

Die vorliegende Erfindung betrifft eine Vorrichtung zur Bestimmung des Füllstandes in einer Karpule gemäß dem Oberbegriff des Patentanspruches 1.

Aus dem Stand der Technik sind Karpulenhalter mit einer integrierten Füllstandsmessung auf dem kapazitiven Messprinzip bekannt. Dabei wird mittels auf einer Folie angebrachten Elektroden der aktuelle Füllstand der Karpule über den Wechsel des Dielektrikums ähnlich einem Plattenkondensator gemessen. Die Karpulen beinhalten unterschiedliche Flüssigkeiten und werden beispielsweise bei der Verabreichung von spezifischen Dosen von Insulin mittels einer an den Karpulenhalter angebrachten Injektionsnadel oder Spritze angewandt. Bei den bekannten Vorrichtungen wird die Kapazitätsänderung in der Injektionslösung bzw. zwischen der Injektionslösung und der Luft gemessen. Die Kapazität ist neben dem Dielektrikum auch von der Elektrodenfläche und des Elektrodenabstands abhängig. Übliche in den Karpulenhalter eingesetzte Karpulen weisen Tolleranzen auf, die insbesondere im Durchmesser von ± 0,06 mm liegen. Um das Einschieben verschiedener Karpulen in den Karpulenhalter zu ermöglichen, muss dieser diese Tolleranzen im Durchmesser zulassen. Die Elektroden werden dadurch bei dickeren Karpulen sehr fest an der Karpulenwand angepresst bzw. liegen bei dünnen Karpulen locker zwischen Karpulenhalter und Karpule, dadurch werden beim Austausch von Karpulen und Lageänderungen bei gleichem Füllstand verschiedene Messergebnisse in Bereichen von 10 µl erfasst. Dies entspricht beispielsweise bei Insulin ca. 8 Injektionseinheiten. Besonders bei schnell wirkenden und hochdosierten Medikamenten werden nur einige wenige µl bei einer Verabreichung gespritzt, wodurch jede geringe Fehlmessung zu einer falschen Erfassung der Gesamtverabreichung beispielsweise über einen Tag verteilt gemessen wird oder Fehler in der Erfassung der Restmenge in der Karpule entstehen.

Aufgabe der Erfindung ist es daher die Fehler in der Füllstandsmessung zu reduzieren und ein zuverlässiges System zur Füllstandsmessung in Karpulen bereitzustellen.

Diese Aufgabe wird durch die kennzeichnenden Merkmale des Anspruches 1 gelöst. Dabei ist vorgesehen, dass der Karpulenhalter im Bereich zumindest einer Elektrode einen, insbesondere vorgespannten, flexiblen Bereich und/oder ein flexibles Element aufweist, wobei der flexible Bereich oder das flexible Element die Folie, zumindest im Bereich der Elektrode, in Richtung der Karpule andrückt.

Durch die flexibeln Bereiche und/oder die flexiblen Elemente wird eine Anpressung der Elektrode an eine Karpule erreicht und die Füllstandmessung verbessert. Zusätzlich werden die Durchmesserunterschiede und Fertigungstoleranzen ausgeglichen und die Messgenauigkeit erhöht.

Besonders vorteilhafte Ausführungsformen der Vorrichtung werden durch die Merkmale der abhängigen Ansprüche näher definiert:
So kann die Messgenauigkeit weiter erhöht werden, wenn die Folie im Zwischenbereich zwischen dem Karpulenhalter und der Karpule angeordnet ist und am Außenbereich der Karpule flächig anliegt.

Vorteilhafte Ausführungsformen der Erfindung werden bereitgestellt, indem die Ausnehmung des Karpulenhalters den gleichen oder einen geringeren Innendurchmesser als der Außendurchmesser der Karpule aufweist, wobei der Durchmesserunterschied durch den flexiblen Bereich und/oder das flexible Element bei Einsetzen der Karpule in den Karpulenhalter, insbesondere durch Dehnung oder Verformung des Werkstoffs des Karpulenhalters, vorzugsweise im flexiblen Bereich, und/oder des flexiblen Elements ausgleichbar ist und der Karpulenhalter zumindest im flexiblen Bereich und/oder dem flexiblen Element über der Folie, insbesondere über der Elektrode, an der Karpule anliegt.

Eine bevorzugte Ausbildung des flexiblen Bereichs wird bereitgestellt, wenn der flexible Bereich des Karpulenhalters eine gegenüber dem Rest des Karpulenhalters verdünnte Wandstärke und/oder eine erhöhte Elastizität aufweist. Dadurch kann auch auf kostengünstige Weise der Karpulenhalter im 3-D Druckverfahren, in Druck- oder Spritzguss aus einem Kunststoff hergestellt werden.

Die Anpressung der Elektrode an die Karpule kann weiter verbessert werden, wenn der flexible Bereich ziehharmonikaförmig, gefalten, krallenförmig oder wulstförmig ausgebildet ist.

Der flexible Bereich bzw. der Karpulenhalter kann einfach hergestellt werden, wenn der Karpulenhalter im flexiblen Bereich zumindest eine Haupt-Freistellung aufweist, die derart angeordnet ist, dass zumindest ein, insbesondere in die Ausnehmung des Karpulenhalter hinein ragendes, in radialer Richtung flexibles Federelement ausgebildet ist. Weiters kann die Wirkung der Anpressung erhöht werden, wenn an beiden Enden des Federelements entlang eines Teils des Umfangs des Karpulenhalters verlaufende Neben-Freistellungen angeordnet sind, wobei die Neben-Freistellungen das Federelement an dessen Stirnseiten begrenzen.

Eine vorteilhafte Ausführungsform der erfindungsgemäßen Vorrichtung wird bereitgestellt, wenn an der Haupt-Freistellung zwei, insbesondere gleich ausgebildete, Federelemente einander gegenüber angeordnet sind, wobei insbesondere die Haupt-Freistellung entlang der Achse des Karpulenhalters verläuft.

Die Anpressung mehrerer Elektroden oder die Anpressung in mehreren Bereichen entlang des Karpulenhalters kann vorteilhaft erreicht werden, wenn über die gesamte Länge der Karpule eine Anzahl von flexiblen Bereichen und/oder flexiblen Elementen angeordnet sind, wobei die flexiblen Bereiche und/oder die flexiblen Elemente entlang eines Teils, insbesondere entlang der gesamten Länge, des Karpulenhalters angeordnet sind.

Eine weitere vorteilhafte Ausführungsform der Vorrichtung wir bereitgestellt, indem das flexible Element zwischen dem Karpulenhalter und der Folie angeordnet ist, wobei das flexible Element kompressibel ausgebildet ist und derart ausgebildet ist, dass das flexible Element durch elastische Verformung die Folie, zumindest im Bereich der Elektrode, in Richtung der Karpule andrückt.

Weitere Vorteile und Ausgestaltungen der Erfindung ergeben sich aus der Beschreibung und den beiliegenden Zeichnungen.

Die Erfindung ist im Folgenden anhand von besonders vorteilhaften, aber nicht einschränkend zu verstehenden Ausführungsbeispielen in den Zeichnungen schematisch dargestellt und wird unter Bezugnahme auf die Zeichnungen beispielhaft beschrieben.

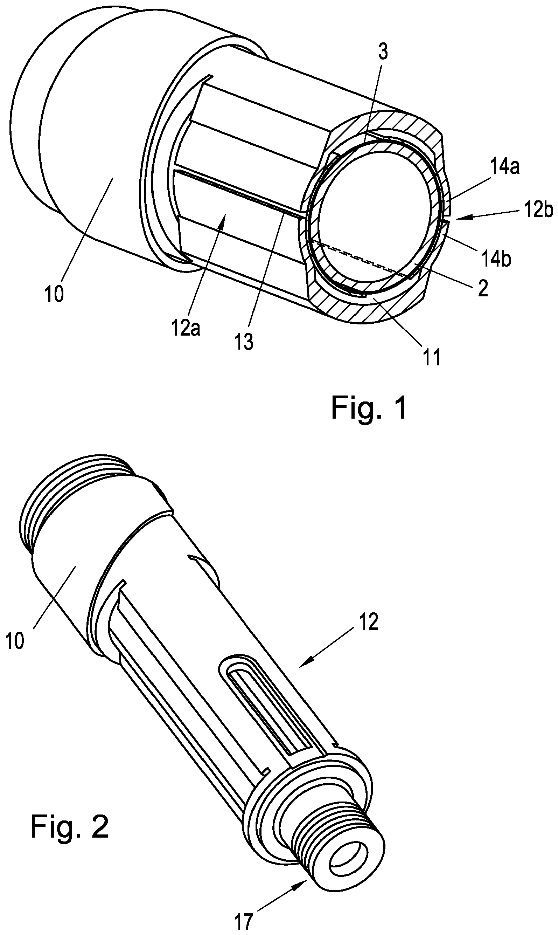

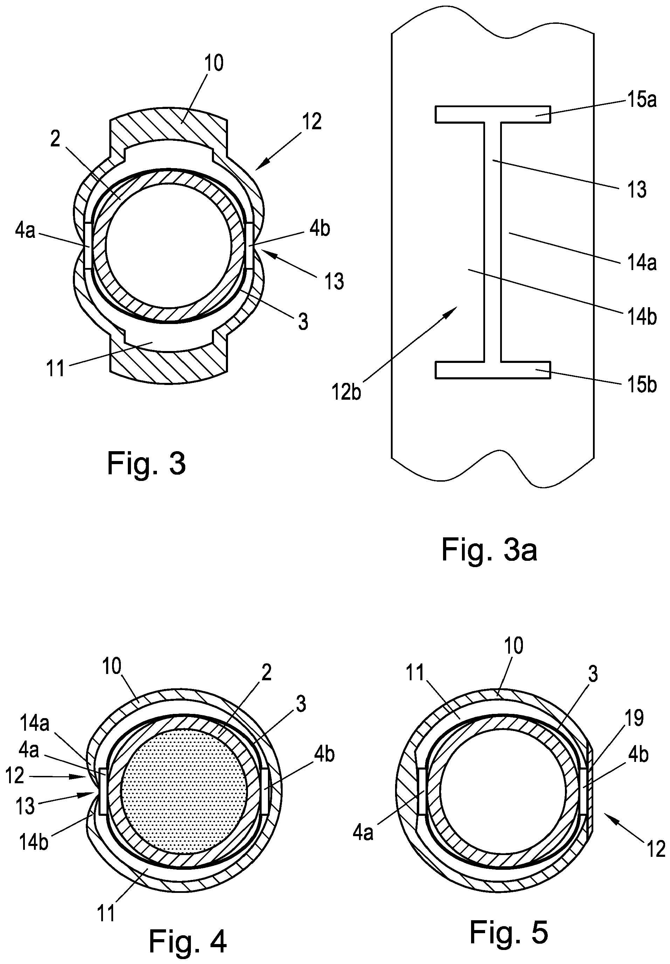

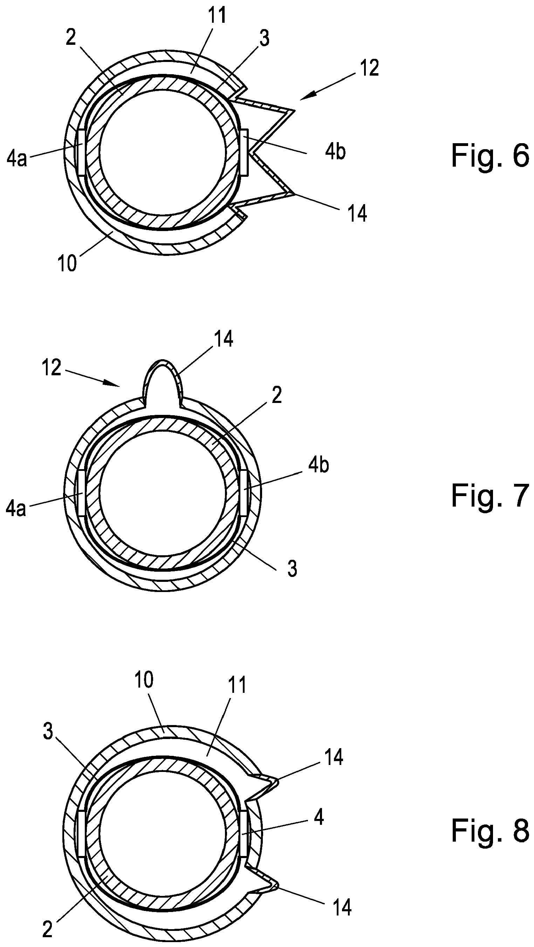

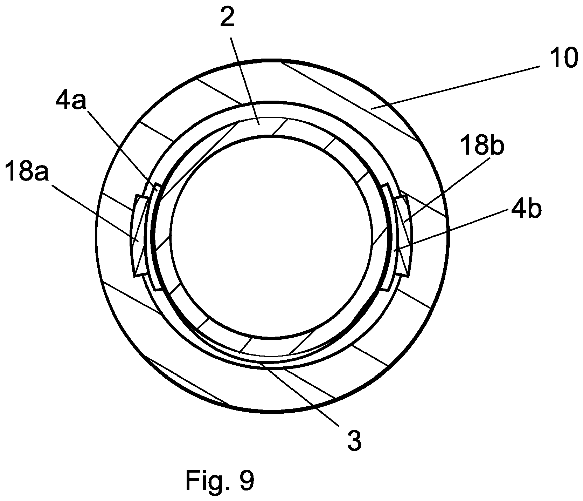

Fig. 1 zeigt eine perspektivische Schnittansicht einer erfindungsgemäßen Ausführung der Vorrichtung, Fig. 2 zeigt eine perspektivische Ansicht der Vorrichtung gemäß Fig. 1, Fig. 3 zeigt eine Schnittansicht der Ausführungsform gemäß Fig. 1, Fig. 3a zeigt eine Detailansicht der Vorrichtung gemäß Fig. 1, Fig. 4 zeigt eine zweite Ausführungsform der erfindungsgemäßen Vorrichtung, Fig. 5 zeigt eine dritte Ausführungsform der erfindungsgemäßen Vorrichtung, Fig. 6 zeigt eine vierte Ausführungsform der erfindungsgemäßen Vorrichtung, Fig. 7 zeigt eine fünfte Ausführungsform der erfindungsgemäßen Vorrichtung, Fig. 8 zeigt eine sechste Ausführungsform der Erfindung und Fig. 9 eine siebte Ausführungsform der erfindungsgemäßen Vorrichtung.

Fig. 1 zeigt eine perspektivische Ansicht der erfindungsgemäßen Vorrichtung zur Bestimmung des Füllstandes in einer Karpule 2 in einer Schnittansicht, wobei der Schnitt normal zur Achse der Karpule 2 verläuft. Die Vorrichtung umfasst einen Karpulenhalter 10, der eine Ausnehmung 11 aufweist, in die die Karpule 2 eingebracht ist. Im Zwischenbereich zwischen der Wandung des Karpulenhalters 10 und der Karpule 2 ist in der Ausnehmung 11 eine Folie 3 angeordnet. Auf oder in der Folie 3 sind zwei Elektroden 4a und 4b angeordnet, die in dieser Abbildung nicht dargestellt sind. Mittels der auf der Folie 3 angebrachten Elektroden 4a und 4b wird der Füllstand der Karpule 2 bzw. der in der Karpule 2 befindlichen Flüssigkeit gemessen. Der Karpulenhalter 10 weist einen ersten flexiblen Bereich 12a auf, der die auf der Folie 3 unter dem flexiblen Bereich 12a angeordnete Elektrode 4a an die Wandung der Karpule 2 andrückt. Im flexiblen Bereich 12a weist der Karpulenhalter 10 eine Hauptfreistellung 13 auf, die parallel zum Verlauf der Achse der Karpule 2 bzw. der Achse der Ausnehmung 11 verläuft und im flexiblen Bereich 12a die Wandung des Karpulenhalters 10 vollständig durchsetzt. Die Hauptfreistellung 13 teilt den flexiblen Bereich 12a bzw. die Wandung des Karpulenhalters 10 im flexiblen Bereich 12a in zwei Federelemente 14a und 14b. Die Federelemente 14a und 14b sind dabei vorgespannt und werden bei Einsetzen der Karpule 2 in radialer Richtung zur Ausnehmung 11 bzw. Karpule 2 nach außen gedrückt, wodurch die Folie 3 und die auf der Folie 3 angeordneten Elektroden 4a bzw. 4b an der Karpule 2 angedrückt werden. Diametral gegenüber in Bezug auf die Achse des Karpulenhalters 10 ist dem flexiblen Bereich 12 ein weiterer gleichartig ausgebildeter flexibler Bereich 12b angeordnet, der die unter diesem flexiblen Bereich 12b befindliche Elektrode 4b an die Karpule 2 andrückt.

Fig. 2 zeigt eine perspektivische Ansicht der ersten Ausführungsform gemäß Fig. 1. An der Spitze des Karpulenhalters 10 ist ein Anschlussbereich 17 ausgebildet, an dem Verabreichungsmittel, wie z.B. Spritzen- oder Katheteranschlüsse, angeordnet sein können.

Fig. 3 zeigt eine Schnittansicht der erfindungsgemäßen Vorrichtung nach Fig. 1. Der Schnitt ist senkrecht zur Achse des Karpulenhalters 10 bzw. der Ausnehmung 11 bzw. senkrecht zur Rotationsachse der kreisrund ausgebildeten Karpule 2 dargestellt. An der Außenfläche der Karpule 2 liegt die Folie 3 mit den Elektroden 4a und 4b an der Karpule 2 an, wobei die flexiblen Bereiche 12a und 12b die Folie 3 im Bereich der Elektroden 4a und 4b an die Karpule 2 andrücken. Die flexiblen Bereiche 12a und 12b sind gleichartig ausgebildet und weisen eine Hauptfreistellung 13 auf, die parallel zur Achse des Karpulenhalters 2 bzw. zur Achse der Karpule verläuft. In den flexiblen Bereichen 12a bzw. 12b ist die Wandstärke des Karpulenhalters 10 verdünnt ausgeführt und weist eine erhöhte Elastizität auf.

Fig. 3a zeigt eine schematische Detailansicht des flexiblen Bereichs 12b gemäß Fig. 3. Durch die Hauptfreistellung 13 werden im flexiblen Bereich 12b zwei einander gegenüberliegende Federelemente 14a, 14b ausgebildet. An der Stirnseite jedes Federelements 14a, 14b befindet sich eine Nebenfreistellung 15a bzw. 15b, die jeweils am Ende der Hauptfreistellung 13 senkrecht zu dieser verläuft. Die Nebenfreistellung 15a bzw. 15b begrenzen die jeweiligen Federelemente 14a bzw. 14b. An dem der Hauptfreistellung 13 gegenüberliegenden Ende der Federelemente 14a bzw. 14b sind diese mit dem Material des Karpulenhalters 10 verbunden bzw. setzt sich das Material des Karpulenhalters 10 in den Federelementen 14a und 14b fort. Die Federelemente 14a und 14b können wie in Fig. 3 bzw. Fig. 1 gezeigt eine dünnere Wandstärke als die restlichen Bereiche des Karpulenhalters 10 oder eine gleiche Wandstärke wie der Karpulenhalter 10 aufweisen. Die Federelemente 14a und 14b können dabei in die Ausnehmung 11 des Karpulenhalters 10 hineinragen und in radialer Richtung zur Achse des Karpulenhalters bzw. zur Achse der Ausnehmung 11 des Karpulenhalters 10 flexibel ausgebildet sein. Bei Einsetzen der Karpule 2 in die Ausnehmung 11 des Karpulenhalters 10 wird ein oder alle Federelemente 14a, 14b in radialer Richtung elastisch verformt, wodurch die zwischen der Karpule 2 und dem Karpulenhalter 10 bzw. den Wandungen des flexiblen Bereichs 12b angeordnete Folie 3 an die Karpule 2 angedrückt wird.

Fig. 4 zeigt eine zweite Ausführungsform der erfindungsgemäßen Vorrichtung in einer Schnittansicht. Bei dieser Ausführungsform ist ein flexibler Bereich 12 an dem Karpulenhalter 10 ausgebildet, wobei der flexible Bereich 12 wie Ausführungsform eins eine Hauptfreistellung 13 aufweist, die parallel zur Achse des Karpulenhalters 10 verläuft. Die Hauptfreistellung 13 erzeugt im flexiblen Bereich zwei einander gegenüber liegende Federelemente 14a und 14b die krallenartig in das Innere des Karpulenhalters bzw. der Ausnehmung 11 des Karpulenhalters 10 hineinragen. Die Federelemente 14a, 14b bzw. die Spitzen der Federelemente 14a und 14b liegen an der ersten Elektrode 4a der Folie 3 an und bewirken mit der gegenüberliegenden Seite des Karpulenhalters 10 im Bereich der Elektrode 4b die Anpressung der beiden Elektroden 4a, 4b an die Karpule 2.

Fig. 5 zeigt eine dritte Ausführungsform der erfindungsgemäßen Vorrichtung in einer Schnittansicht. In die Ausnehmung 11 des Karpulenhalters 10 ist die Karpule 2 eingesetzt. Im flexiblen Bereich 12 des Karpulenhalters 10 ist ein flexibles dehnbares Element 19, beispielsweise ein Kautschukband eingesetzt, das die Erweiterung im Bereich des flexiblen Bereiches 12 des Karpulenhalters 10 erlaubt. Der Abstand des flexiblen Elements 19 von der gegenüberliegenden Wand des Karpulenhalters 10 ist dabei im Ausgangszustand, also ohne eingesetzte Karpule 2, geringer als der Durchmesser der Karpule 2. Bei Einsetzen der Karpule 2 mit der Folie 3 wird im flexiblen Bereich 12 der Karpulenhalter 10 aufgeweitet und die Elektroden 4a, 4b bzw. die Folie an die Karpule 2 angedrückt.

Fig. 6 zeigt eine vierte Ausführungsform der erfindungsgemäßen Vorrichtung in Schnittansicht. Im flexiblen Bereich 12 ist ein ziehharmonikaförmig ausgebildetes Federelement 14 angeordnet. Der Durchmesser im Ausgangszustand des Karpulenhalters 10 bzw. der Außendurchmesser der Ausnehmung 11 des Karpulenhalters 10 ist geringfügig geringer als der Außendurchmesser der Karpule 2. Bei Einsetzen der Karpule 2 mit der darauf angelegten Folie 3 wird der Karpulenhalter 10 im flexiblen Bereich 12 aufgeweitet, das ziehharmonikaförmige elastische Element 19 wird gedehnt bzw. verändert den Winkel zwischen den einzelnen Teilelementen und die Karpule 2 kann in den Karpulenhalter 10 eingebracht bzw. eingeschoben werden. Durch die Aufweitung der Wandung des Karpulenhalters 10 erfolgt eine Anpressung der Folie 3, insbesondere im Bereich der Elektroden 4a, 4b an die Karpule 2.

Fig. 7 zeigt eine fünfte Ausführungsform der erfindungsgemäßen Vorrichtung, wobei im flexiblen Bereich 12 ein wulstförmig nach außen ausgestülptes Federelement 14 angeordnet ist. Das Federelement 14 kann dabei aus einem elastischem Material, wie z.B. Gummi oder anderen elastischen aus dem Stand der Technik bekannten Materialien sein und bewirkt eine Aufweitung des Durchmessers der Ausnehmung 11 bei Einsetzen der Karpule 2 in die Ausnehmung 11. Das Federelement 14 kann dabei vorgespannt ausgebildet sein und die eine Zugkraft im Umfangbereich des Karpulenhalters 10 bewirken, wodurch die Enden des Federelements 14 zueinander gezogen werden. Durch Bestimmung der Vorspannung und eine geeignete Materialauswahl kann so die Zugkraft und damit die Anpressung der Elektroden 4a und 4b an die Karpule 2 definiert werden.

Fig. 8 zeigt eine sechste Ausführungsform der erfindungsgemäßen Vorrichtung in Schnittansicht. Analog zur Ausführung der Fig. 7 ist es möglich zwei wulstartige Federelemente 14a und 14b im flexiblen Bereich 12 des Karpulenhalters 10 anzuordnen. Alternativ können wie in Fig. 8 dargestellt die zwei wulstförmigen Federelemente 14 mittels eines Steges verbunden sein.

Alternativ zu den in den Fig. 1 bis 8 dargestellten Ausführungsformen der Erfindung sind auch Kombinationen von den gezeigten Ausführungsformen möglich. Alternativ können auch bei den gezeigten Ausführungsformen jeweils ein flexibler Bereich 12, zwei oder mehrere flexible Bereiche 12a, 12b, ... vorgesehen sein.

Das Material bzw. der Werkstoff des Karpulenhalters 10 kann im flexiblen Bereich 12 abweichend zu den restlichen Karpulenhalter 10 auch aus einem anderen Material bestehen, wobei bevorzugt elastische Materialien, wie Gummi oder elastische Kunststoffe vorgesehen sind. Ebenso kann das Federelement 14 oder die Federelemente 14a, 14b aus flexiblen oder starren Materialien ausgebildet sein, wobei hier auch bevorzugt Gummi oder elastische Kunststoffe, dünne Metallplättchen, oder andere Federwerkstoffe bevorzugt vorgesehen sind.

Alternativ zu den in den Fig. 1 bis 8 gezeigten Ausführungsformen der Erfindung ist es möglich, dass entlang der Länge des Karpulenhalters 10 mehrere flexible Bereiche über den Umfang verteilt vorgesehen sind, wobei diese flexiblen Bereiche gleichartig oder abweichend zueinander ausgebildet sein können. Es können weiters über den Umfang mehrfach verteilte flexible Bereiche angeordnet sein und zusätzlich oder alternativ entlang der Achse des Karpulenhalters 10 mehrere flexible Bereiche vorgesehen sein.

Fig. 9 zeigt eine siebte Ausführungsform des erfindungsgemäßen Karpulenhalters 10 in einer Schnittansicht. Zwischen der in die Ausnehmung 11 eingebrachten Karpule 2 und dem Karpulenhalter 10 ist die Folie 3 mit den Elektroden 4a, 4b eingebracht. An dem Karpulenhalter 10 bzw. dessen Wandung sind zwei flexible Elemente 18a, 18b diametral gegenüber in der Ausnehmung 11 angeordnet. Zwischen den flexibeln Elementen 18a, 18b und der Karpule 2 sind die Elektroden 4a, 4b, die auf der Folie 3 aufgebracht sind, angeordnet. Die flexiblen Elemente 18 sind kompressibel oder elastisch verformbar und werden bei Einbringung der Karpule 2 in die Ausnehmung 11 des Karpulenhalters 10 komprimiert und drücken durch die elastische Verformung die Folie 3 und/oder die Elektroden 4a, 4b an die Karpule 2 an. Die flexiblen Elemente18 können beispielsweise als flexibles Klebeband ausgebildet sein und an dem Karpulenhalter 10 angeklebt sein oder beispielweise aus einem geschäumten Material in dem Material des Karpulenhalters 10 eingebettet oder an dessen Innenseite angebracht sein.

Alternativ zu der in den Fig. 9 gezeigten Ausführungsform der Erfindung ist es möglich, dass entlang der Länge des Karpulenhalters 10 mehrere flexible Elemente über den Umfang verteilt vorgesehen sind, wobei diese flexiblen Elemente gleichartig oder abweichend zueinander ausgebildet sein können. Es können weiters über den Umfang mehrfach verteilte flexible Elemente angeordnet sein und zusätzlich oder alternativ entlang der Achse des Karpulenhalters 10 mehrere flexible Elemente vorgesehen sein.

Weiters können die Ausführungsformen der Fig. 1 bis 8 mit Ausführungsformen der Fig. 9 kombiniert werden und beispielsweise unter dem flexiblen Bereich 12 ein flexibles Element 18 zur Verstärkung der Anpressung der Folie 3 bzw. der Elektroden 4a, 4b angeordnet sein.

Alternativ bzw. ergänzend zu den gezeigten Ausführungsformen kann die Folie 3 auch aus mehren Teilfolien bestehen oder mehrere Folien 3 vorgesehen sein und/oder die Elektroden 4a, 4b auch jeweils auf den Teilfolien oder den unterschiedlichen Folien angeordnet sein. Die jeweiligen Folien oder Teilfolien werden dabei jeweils im Bereich der flexibeln Bereiche 12a, 12b,... angeordnet. Weiters kann die Folie 3, die Teilfolien oder auch die mehreren Folien auch steifere und/oder flexiblere Abschnitte aufweisen und über deren Breite oder Länge unterschiedliche Steifigkeiten oder Dicken aufweisen.

Die Folie 3 kann bei den beschriebenen Ausführungsformen auch als Flexprint bzw. in einem Flexprintverfahren ausgebildet sein und die Elektrode 4 oder Elektroden 4a, 4b sowie die jeweiligen Leiterbahnen auf diesem Flexprint aufgedruckt, geätzt oder in die Folie 3 bzw. Folien eingebettet sein. Ein bevorzugter Werkstoff für die Folie ist dabei Polyimid mit Kupferbeschichtung, wobei auch andere Kunststoffe oder geeignete Folienwerkstoffe verwendet werden können.

## Patentansprüche

1. Vorrichtung zur Bestimmung des Füllstandes in einer Karpule (2), wobei der Füllstand mittels zumindest einer auf zumindest, insbesondere genau, einer Folie (3) angebrachten Elektrode (4) messbar ist, umfassend:
einen Karpulenhalter (10), der eine Ausnehmung (11) aufweist, in die die Karpule (2) eingebracht ist,
- wobei die Folie (3) im Zwischenbereich zwischen dem Karpulenhalter (10) und der Karpule (2) oder im Werkstoff des Karpulenhalters (10) angeordnet ist,
**dadurch gekennzeichnet, dass**
der Karpulenhalter (10) im Bereich zumindest einer Elektrode (4) einen, insbesondere vorgespannten, flexiblen Bereich (12) und/oder ein flexibles Element (18) aufweist, wobei der flexible Bereich (12) oder das flexible Element (18) die Folie (3), zumindest im Bereich der Elektrode (4), in Richtung der Karpule (2) andrückt.

2. Vorrichtung nach Anspruch 1, **dadurch gekennzeichnet, dass** die Folie (3) im Zwischenbereich zwischen dem Karpulenhalter (10) und der Karpule (2) angeordnet ist und am Außenbereich der Karpule (2) flächig anliegt.

3. Vorrichtung nach einem der vorangehenden Ansprüche, **dadurch gekennzeichnet, dass** die Ausnehmung (11) des Karpulenhalters (10) den gleichen oder einen geringeren Innendurchmesser als der Außendurchmesser der Karpule (2) aufweist, wobei der Durchmesserunterschied durch den flexiblen Bereich (12) und/oder das flexible Element (18) bei Einsetzen der Karpule (2) in den Karpulenhalter (10), insbesondere durch Dehnung oder Verformung des Werkstoffs des Karpulenhalters (10), vorzugsweise im flexiblen Bereich (12), und/oder des flexiblen Elements (18) ausgleichbar ist und der Karpulenhalter (10) zumindest im flexiblen Bereich (12) und/oder dem flexiblen Element (18) über der Folie (3), insbesondere über der Elektrode (4), an der Karpule (2) anliegt.

4. Vorrichtung nach einem der vorangehenden Ansprüche, **dadurch gekennzeichnet, dass** der flexible Bereich (12) des Karpulenhalters (10) eine gegenüber dem Rest des Karpulenhalters (10) verdünnte Wandstärke und/oder eine erhöhte Elastizität aufweist.

5. Vorrichtung nach einem der vorangehenden Ansprüche, **dadurch gekennzeichnet, dass** der flexible Bereich (12) ziehharmonikaförmig, gefalten, krallenförmig oder wulstförmig ausgebildet ist.

6. Vorrichtung nach einem der vorangehenden Ansprüche, **dadurch gekennzeichnet, dass** der Karpulenhalter (10) im flexiblen Bereich (12) zumindest eine Haupt-Freistellung (13) aufweist, die derart angeordnet ist, dass zumindest ein, insbesondere in die Ausnehmung (11) des Karpulenhalter (10) hinein ragendes, in radialer Richtung flexibles Federelement (14) ausgebildet ist.

7. Vorrichtung nach Anspruch 6, **dadurch gekennzeichnet, dass** an beiden Enden des Federelements (14) entlang eines Teils des Umfangs des Karpulenhalters (10) verlaufende Neben-Freistellungen (15) angeordnet sind, wobei die Neben-Freistellungen (15) das Federelement (14) an dessen Stirnseiten begrenzen.

8. Vorrichtung nach einem der Ansprüche 6 oder 7, **dadurch gekennzeichnet, dass** an der Haupt-Freistellung (13) zwei, insbesondere gleich ausgebildete, Federelemente (14a, 14b) einander gegenüber angeordnet sind, wobei insbesondere die Haupt-Freistellung (13) entlang der Achse des Karpulenhalters (10) verläuft.

9. Vorrichtung nach einem der vorangehenden Ansprüche, **dadurch gekennzeichnet, dass** über die gesamte Länge der Karpule (2) eine Anzahl von flexiblen Bereichen (12) und/oder flexiblen Elementen (18) angeordnet sind, wobei die flexiblen Bereiche (12) und/oder die flexiblen Elemente (18) entlang eines Teils, insbesondere entlang der gesamten Länge, des Karpulenhalters (10) angeordnet sind.

10. Vorrichtung nach einem der vorangehenden Ansprüche, **dadurch gekennzeichnet, dass** das flexible Element (18) zwischen dem Karpulenhalter (10) und der Folie (3) angeordnet ist, wobei das flexible Element (18) kompressibel ausgebildet ist und derart ausgebildet ist, dass das flexible Element (18) durch elastische Verformung die Folie (3), zumindest im Bereich der Elektrode (4), in Richtung der Karpule (2) andrückt.
